# EUROPÄISCHE PATENTANMELDUNG

(11) **EP 3 199 156 A1**
(43) Veröffentlichungstag der Anmeldung: **02.08.2017**
(21) Anmeldenummer: 16000233.3
(22) Anmeldetag: 01.02.2016
(51) Int. Cl.: A61K 31/496

(54) **VERWENDUNG VON GLYCIN-TRANSPORTER-1-ANTAGONISTEN ALS ZENTRALNERVÖSE ANALGETIKA**

(71) Anmelder: Heinrich Heine Universität Düsseldorf, 40225 Düsseldorf (DE); Friedrich-Alexander-Universität Erlangen-Nürnberg, 91054 Erlangen (DE)
(72) Erfinder: Werdehausen, Robert, 40225 Düsseldorf (DE); Hermanns, Henning, 1077 ZH Amsterdam (NL); Eulenburg, Volker, 91088 Bubenreuth (DE)
(74) Vertreter: Sparing, Rolf Klaus

(57) **Zusammenfassung**

Die Erfindung betrifft die Verwendung von Verbindungen der Formel (I) als Glycin-Transporter-1-Antagonisten zur Behandlung von chronischen und/oder neuropathischen Schmerzen.

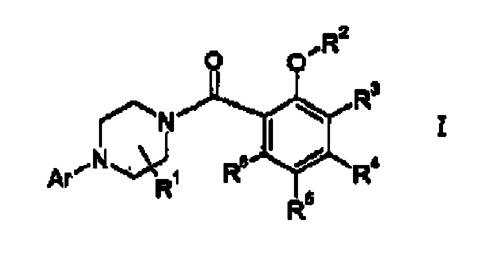

## Beschreibung

Die Erfindung betrifft die Verwendung von Verbindungen der Formel (I) als Glycin-Transporter-1-Antagonist zur Behandlung chronischer und/oder neuropathischer Schmerzen.

Die Behandlung von chronischen bzw. neuropathischen Schmerzen ist eine Herausforderung für Ärzte und Patienten, da es bislang keine Medikamente gibt, die speziell zur Behandlung chronischer Schmerzen wie z.B. Neuropathien ausgebildet sind. Die bislang bekannten Medikamente zeigen gegenwärtig sehr häufig eine unzureichende Linderung und offenbaren häufig unerwünschte Nebenwirkungen wie Müdigkeit, Konzentrationsstörungen, Schwindel, Kopfschmerzen, Übelkeit und Toleranzentwicklung. Zusätzlich zeigen einige der gegenwärtig eingesetzten Medikamente ein Abhängigkeitspotential.

Glycin-abhängige Neurotransmission trägt entscheidend zur Modulation der Schmerzwahrnehmung bei und Dysfunktionen tragen wesentlich zur Ontogenese und Chronifizierung von Schmerzen bei. Eine pharmakologische Verstärkung der glycinergen Inhibition stellt demnach einen vielversprechenden therapeutischen Ansatz dar. Die Glycinkonzentration im synaptischen Spalt wird durch die Glycintransporter GlyT1 und GlyT2 reguliert. Bisher untersuchte Inhibitoren von GlyT, insbesondere von GlyT1, können nozizeptives Verhalten reduzieren, weisen aber auch erhebliche unerwünschte Wirkungen auf und wirken irreversibel. Chronische Schmerzen, z.B. verursacht durch eine Neuropathie sind eine zunehmende Belastung für die Gesellschaft und verursachen hohe Kosten in der Gesundheitsversorgung und führen zu erheblichen Produktivitätsverlusten. Momentan gibt es keine ausreichend wirksamen und verträglichen Therapien zur Linderung von chronischen und/oder neuropathischen Schmerzen. Es besteht daher ein dringender Bedarf an Medikamenten, die chronische und/oder neuropathische Schmerzen lindern können.

Aufgabe der Erfindung ist daher die Bereitstellung eines Medikaments zur Behandlung chronischer und/oder neuropathischer Schmerzen, das keine relevanten unerwünschten Wirkungen aufweist.

Die Aufgabe der Erfindung wird vorliegend durch die kennzeichnenden Merkmale der Ansprüche 1 und 10 gelöst.

Die vorliegende Erfindung umfasst die Verwendung eines Glycin-Transporter-1-Antagonisten in der Herstellung eines Medikaments zur Behandlung chronischer und/oder neuropathischer Schmerzen nach der allgemeinen Formel I, 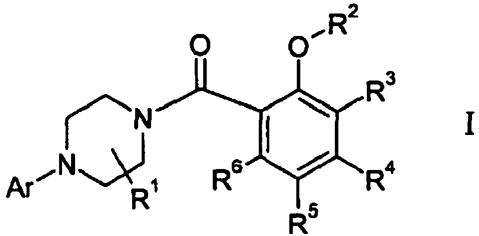
worin Ar unsubstituiertes oder substituiertes Aryl, ausgewählt aus der Gruppe, bestehend aus Phenyl, Benzyl, Naphthyl, Biphenyl und Indanyl, oder 6-gliedriges Heteroaryl ist, enthaltend ein, zwei oder drei Stickstoffatome, und wobei die Aryl- und die Heteroarylgruppen durch einen oder mehrere Substituenten, ausgewählt aus der Gruppe, bestehend aus Hydroxy, Halogen, NO₂, CN, (C₁-C₆)-Alkyl, (C₁-C₆)-Alkyl, substituiert durch Halogen, (C₁-C₆)-Alkyl, substituiert durch Hydroxy, (CH₂)ₙ-(C₁-C₆)-Alkoxy, (C₁-C₆)-Alkoxy, substituiert durch Halogen, NR₇R₈, C(O)R₉, So₂R₁₀,-C(CH₃)=NOR₇, oder durch einen 5-gliedrigen aromatischen Heterozyklus, enthaltend 1 bis 4 Heteroatome, ausgewählt aus N und O, der gegebenenfalls durch (C₁-C₆)-Alkyl substituiert ist, substituiert sein können;
R₁ = Wasserstoff oder (C₁-C₆)-Alkyl ist;
R₂ = Wasserstoff, (C₁-C₆)-Alkyl, (C₂-C₆)-Alkenyl, (C₁-C₆)-Alkyl, substituiert durch Halogen, (C₁-C₆)-Alkyl, substituiert durch Hydroxy, (CH₂)ₙ-(C₃-C₇)-Cycloalkyl, gegebenenfalls substituiert durch (C₁-C₆)-Alkoxy oder durch Halogen, ist oder CH(CH₃)-(C₃-C₇)-Cycloalky), (CH₂)ₙ₊₁-C(O)-R_{g}, (CH₂)ₙ₊₁-CN, Bicyclo [2.2.1]heptyl, (CH₂)ₙ₊₁-O-(C₁-C₆)-Alkyl, (CH₂)ₙ-Heterocycloalkyl, (CH₂)ₙ-Aryl oder (CH₂)ₙ-Heteroaryl mit 5 oder 6 Ringgliedern, enthaltend ein, zwei oder drei Heteroatome, ausgewählt aus der Gruppe, bestehend aus Sauerstoff, Schwefel oder Stickstoff, ist, worin Aryl, Heterocycloalkyl und Heteroaryl unsubstituiert oder durch einen oder mehrere Substituenten substituiert sind, ausgewählt aus der Gruppe, bestehend aus Hydroxy, Halogen, (C₁-C₆)-Alkyl oder (C₁-C₆)-Alkoxy;
R₃, R₄ und R₆ unabhängig voneinander = Wasserstoff, Hydroxy, Halogen, (C₁-C₆)-Alkyl, (C₁-C₆)-Alkoxy oder O-(C₃-C₆)-Cycloalkyl sind;
R₅ = NO₂, CN, C(O)R₉ oder SO₂ R₁₀ ist;
R₇ und R₈ unabhängig voneinander = Wasserstoff oder (C₁-C₆)-Alkyl sind;
R₉ = Wasserstoff, (C₁-C₆)-Alkyl, (C₁-C₆)-Alkoxy oder NR₇R₈ ist;
R₁₀ = (C₁-C₆)-Alkyl, gegebenenfalls substituiert durch Halogen, (CH₂)ₙ-(C₃-C₆)-Cycloalkyl, (CH₂)ₙ-(C₃-C₆)-Alkoxy, (CH₂)ₙ-Heterocycloalkyl oder NR₇R₈ ist;
n 0, 1 oder 2 ist; und Ar zudem substituiertes Phenyl ist, R₂(C₁-C₆)-Alkyl ist und R₅S(O)₂CH₃ oder S(O)₂CH₂CH₃ beinhalten kann, sowie pharmazeutisch akzeptable Säureadditionssalze davon.

Erfindungsgemäß ist vorgesehen, dass die nachfolgenden Verbindungen von der Verwendung für die Herstellung eines Medikaments als Bestandteil der generischen Formel (I) ausgeschlossen sein können:
1-[5-(Aminosulfonyl)-2-methoxybenzoyl]-4-(3-chlorphenyl)-piperazin,
1-[5-(Aminosulfonyl)-2-methoxybenzoyl]-4-(4-fluorphenyl)-piperazin,
1-[5-(Aminosulfonyl)-2-methoxybenzoyl]-4-[3-(trifluormethyl)phenyl]-piperazin,
4-(3-Amino-4-nitrophenyl)-1-[4-(dimethylamino)-2-methoxy-5-nitrobenzoyl]-2-methylpiperazin,
1-[4-(Dimethylamino)-2-methoxy-5-nitrobenzoyl]-2-methyl-4-(4-nitrophenyl)-piperazin,
4-[4-(Dimethylamino)-2-methoxy-5-wtrobenzoyl]-2-methyl-1-(4-nitrophenyl)-piperazin,
1-(2-Chlor-4-nitrophenyl)-4-[4-(dimethylamino)-2-methoxy-5-nitrobenzoyl]-piperazin,
1-[4-(Dimethylamino)-2-methoxy-5-nitrobenzoyl]-4-(2,4-dinitrophenyl)-2-methylpiperazin,
1-(4-Chlor-2-nitrophenyl)-4-[4-(dimethylamino)-2-methoxy-5-nitrobenzoyl]-piperazin,
4-[4-(Dimethylamino)-2-methoxy-5-nitrobenzoyl]-1-(2,4-dinitrophenyl)-2-methylpiperazin und
1-[(2-Benzyloxy-5-formyl)benzoyl]-4-phenylpiperazin.

Insbesondere hat sich aber erfindungsgemäß die Verwendung einer Verbindung der Formel (II) zur Herstellung eines Medikaments zur Behandlung chronischer und/oder neuropathischer Schmerzen als vorteilhaft herausgestellt.

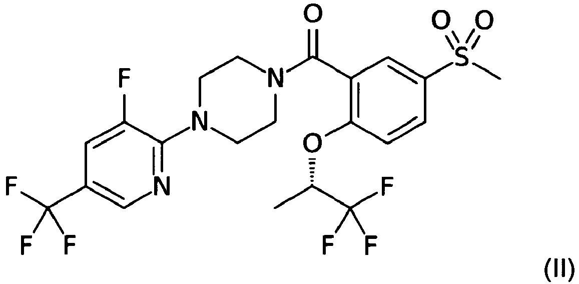

Erfindungsgemäß ist die Verwendung einer Verbindung nach den Formeln (I) oder (II) und eines Verfahrens zur Behandlung chronischer und/oder neuropathischer Schmerzen vorgesehen, umfassend die Verabreichung eines Medikaments beinhaltendend Verbindungen der Formeln (I) oder (II) an ein Individuum in einer therapeutisch wirksamen Menge als Glycin-Transporter-1-Antagonist. Es hat sich dabei herausgestellt, dass eine erfolgreiche Behandlung chronischer und/oder neuropathischer Schmerzen erzielt werden kann ohne dass hierbei relevante unerwünschte Wirkungen auftreten. Dabei hat sich herausgestellt, dass sich insbesondere die Verbindung {4-[3-fluoro-5-(trifluoromethyl)pyridin-2-yl]-piperazin-1-yl}{5-(methylsulfonyl)-2-[(1S)-2,2,2-trifluoro-1-methylethoxy]-phenyl}-methanone} mit dem Handelsnamen Bitopertin als Bestandteil eines Medikaments für eine therapeutische Behandlung chronischer und/oder neuropathischer Schmerzen eignet. In spezifischen Ausführungsformen kann das therapeutische Medikament auch zusätzliche Analgetika, wie beispielsweise Opiate (z.B. Oxycodon), NSAIDs (z.B. Ibuprofen, Diclofenac) und COX-2-Inhibitoren (z.B. Etoricoxib) enthalten.

Gegenüber bereits bekannten GlyT1-Inhibitoren bietet Bitopertin den Vorteil hervorragender oraler Verfügbarkeit des Wirkstoffes und damit eine praktische Einsetzbarkeit bei gleichzeitig sehr guten schmerzlindernden Effekten, ohne dass die typischen zentralnervösen Nebenwirkungen einer irreversiblen und vollständigen GIyT1 Hemmung zu beobachten waren. Diese Beobachtung ist insofern überraschend, als diese Substanz entwickelt wurde, um über eine Erhöhung endogener Glycinkonzentrationen die Funktion des NMDA-Rezeptors zu verbessern und psychotische Krankheiten, wie Schizophrenie zu behandeln.

Im Gegensatz dazu resultiert die Wirkung im Falle chronischer und/oder neuropathischer Schmerzen zumindest zum Teil aus einer Erhöhung der Glycinkonzentration an hemmenden Glycinrezeptoren und bewirkt somit eine Steigerung spinaler Schmerzhemmung bei einem sehr günstigen Nebenwirkungsprofil und guten pharmakologischen Eigenschaften, d.h. reversible Hemmung von GlyT1, hohe orale Verfügbarkeit und günstige Halbwertszeit.

Die Erfindung umfasst auch ein Medikament in Form von Tabletten, Kapseln oder Sachets zur oralen Verabreichung und in Form einer injizierbaren Lösung, wobei auch zwei oder mehr verschiedene Analgetika (z.B. Opiate, NSAIDs und COX-2-Inhibitoren) bzw. Co-Analgetika (z.B. Antikonvulsiva, z.B. Gabapentin oder Antidepressiva, z.B. Amitriptylin) Bestandteile des Medikaments sein können und mindestens eines der Analgetika ein GlyT1-Antagonist nach Formel I oder II ist.

Die in der Beschreibung und den Ansprüchen dieser Anmeldung verwendeten Begriffe haben die folgende Bedeutung:
"Chronischer Schmerz" bezieht sich auf einen lang anhaltenden persistierenden Schmerz, der auch nach der Heilung des für den Schmerz zugrunde liegenden Zustandes fortbestehen kann. In diesem Fall nimmt das Individuum Schmerz wahr, auch wenn keine offensichtliche Schädigung mehr vorliegt. Somit ist chronischer Schmerz ein Schmerz, der über den zu erwartenden Heilungsprozess fortbesteht und mehrere Monate bis Jahre andauern kann. Chronische und/oder neuropathische Schmerzen können bei malignen Erkrankungen (bösartigen Neubildungen) oder durch die damit verbundenen Behandlungen ausgelöst werden und persistent sein. Weitere Ursachen für chronische Schmerzen können degenerative Erkrankungen, entzündliche Gewebeveränderungen, Diabetes mellitus (Zuckerkrankheit), Neuropathie (Nervenerkrankungen) oder Nervenschäden (z.B. durch Verletzungen) sein.

"Neuropathischer Schmerz" bedeutet vorliegend eine zumindest vorübergehende spinale oder supraspinale Reizung des Nervensystems. Dieser äußert sich häufig mit Dauerschmerz, wiederholt und spontan auftretenden Schmerzanfällen, krankhaft übersteigerter Schmerzwahrnehmung (Hyperalgesie) und der schmerzhaften Wahrnehmung von normalerweise nicht schmerzhaften Reizen (Allodynie). Die beschriebenen Schmerzsymptomatiken können als chronischer und/oder neuropathischer Schmerz kumulativ zusammenfallen. Der neuropathische Schmerz kann jedoch auch als singuläre Symptomatik in Rahmen einer kurzfristigen Reizung des gesamten zentralnervösen Systems auftreten ohne bereits die klinischen Kriterien für "chronische Schmerzen" zu erfüllen.

"Verabreichen" bezieht sich auf das Verfahren zur Einführung eines Medikaments in ein zu behandelndes Individuum. Der Verabreichungsweg kann auf den Bedarf des Individuums zugeschnitten werden. Im Allgemeinen erfolgt die Verabreichung topisch, nasal oder oral (enteral). Sie kann aber auch auf systemischem Wege, d.h. durch eine intramuskuläre oder intravenöse (parenterale) Injektion erfolgen. Der Verabreichungsort kann in der Nähe der Position der wahrgenommenen neuropathischen Schmerzen liegen. "Co-verabreichen" bedeutet, dass mit dem Glycin-Transporter-1-Antagonist der Formeln (I) und (II) simultan mindestens ein weiteres Analgetikum verabreicht wird.

"eine Menge, die zur Behandlung von" ist eine Menge (Dosis), die zu einer messbaren Verringerung von neuropathischen Schmerzen führt. Hierbei ist weder eine vollständige Beseitigung der Schmerzen noch eine Permanenz der Reduktion erforderlich.

Das "Individuum", das behandelt werden soll, kann ein Säugetier mit chronischen bzw. neuropathischen Schmerzen oder ein Mensch sein. Jedoch ist die Erfindung auch anwendbar auf andere Säugetiere, insbesondere Tiere wie Hunde, Katzen und Pferde.

"GlyT1-Antagonist" oder "Glycin-Transporter-1-Antagonist" bezeichnet ein Molekül, das den Transport von Glycin über den GlyT1 hemmt.

Die aktuelle Pharmakotherapie von Schmerzen erfolgt über Interaktion mit verschiedensten molekularen Zielstrukturen im peripheren und zentralen Nervensystem, wodurch die Reizweiterleitung der Schmerzempfindung gehemmt wird. Ein solcher Ansatz versagt jedoch, um wirkungsvoll chronische Schmerzen zu behandeln. Es wird daher angenommen, dass chronische und/oder neuropathische Schmerzen anders als andere Schmerzformen wie akute, reflektorische oder psychosomatische Schmerzen eine periphere, spinale sowie supraspinale Komponente aufweisen.

Zusätzlich zu der beanspruchten Verbindung (I) und der spezifischen Verbindung (II) mit dem Handelsnamen Bitopertin könnten auch Medikamente zur Behandlung neuropathischer Schmerzen eingesetzt werden, die pharmazeutisch wirksame Salze dieser Substanz verwenden. Es könnten auch Vorläufer-Verbindungen, die *in vivo* umgewandelt werden (z.B. metabolisiert) in dem Medikament verwendet werden, die modifiziert substituiert sind, wie Salze, Ester und alkylierte Formen der Verbindung (I) und der Verbindung (II).

Das therapeutische Medikament zur Behandlung der chronischen Schmerzen muss in einer ausreichenden Menge vorhanden sein, um wirksam sein zu können. Dabei muss die im Medikament vorhandene wirksame Verbindung so verabreicht werden, dass weder eine Toxizität oder eine Sättigung des Organismus auftritt, noch das Medikament seine Wirksamkeit verliert. Die einzusetzenden Mengen (Dosierungen) sind von verschiedenen Faktoren abhängig, einschließlich der Art der Verabreichung (unmittelbarere Erreichung des zentralen Nervensystems durch Fähigkeit, die Blut-Hirn-Schranke zu passieren = niedrigere Dosis) und Häufigkeit der Verabreichung (kontinuierliche oder eine häufigere Verabreichung = niedrigere Dosis). Es hat sich gezeigt, dass der Anteil der Verbindungen nach den Formeln (I und II) im Medikament einen Bereich von 0,1 bis 10000mg, vorteilhafter Weise 1 bis 1000mg und in besonders vorteilhafter Weise einen Bereich von 10 bis 1000mg umfasst.

Bei dem Verfahren der Erfindung können Kombinationen von zwei oder mehr GlycinTransporter-Antagonisten verwendet werden. Darüber hinaus können zusätzliche Analgetika oder Co-Analgetika in geeigneter Weise in Kombination mit einem Glycin-Transporter-1-Antagonisten in Form der Verbindung (I) und (II) oder einer Kombination davon verwendet werden. Geeignete Analgetika beinhalten z.B. Opiate, NSAIDs und COX-2-Hemmer. Diese Analgetika können in der gleichen oder geringeren Dosierungen als bei herkömmlichen Schmerzbehandlung eingesetzt werden, da die Kombination betreffend die Wirkungen der Behandlungen allein verwendet potenzieren.

Beispiele von Opiaten umfassen Codein, Morphin, Heroin, Hypodromorphon, Oxycodon, Oxymorphon, Hydrocodon, Meperidin, Fentanyl, Methadon, Darvon, Talwin. Beispiele für NSAIDs umfassen ohne Einschränkung Acetylsalicylsäure, Ibuprofen, Naproxen und Nabumeton. Beispiele für COX-2-Inhibitoren umfassen ohne Einschränkung Celecoxib, Etoricoxib, Rofecoxib, Valdecoxib und Lumiracoxib.

Geeignete Co-Analgetika beinhalten z.B. Antikonvulsiva, Antidepressiva und Lokalanästhetika, insbesondere Lidocain oder seine Metaboliten (z.B. N-Ethylglycin). Diese Co-Analgetika können in der gleichen oder geringeren Dosierungen als bei herkömmlichen Schmerzbehandlung eingesetzt werden, da die Kombination betreffend die Wirkungen der Behandlungen allein verwendet potenzieren.

Beispiele von Antikonvulsiva umfassen ohne Einschränkung z.B. Gabapentin und Pregabalin. Beispiele von Antidepressiva umfassen ohne Einschränkung Monoamin-Wiederaufnahmehemmer (z.B. Amitriptylin), Alpha2-Rezeptor Agonisten (z.B. Clonidin), selektive Serotonin Wiederaufnahmehemmer (z.B. Citalopram), Serotonin-Noradrenalin-Reuptake-Hemmer (z.B. Duloxetin),noradrenerge bzw. melatonerge Antidepressiva (z.B. Mirtazapin bzw. Agomelatin. Beispiele für Lokalanästhetika sind Lidocain (auch inklusive der Metabolite z.B. N-Ethylglycin), Ropivacain und Prilocain.

Für die orale Verabreichung können neben Tabletten, Kapseln und Sachets auch flüssige Zusammensetzungen in Wasser oder anderen wässrigen Vehikeln hergestellt werden. Diese umfassen Lösungen, Emulsionen, Sirupe und Elixiere und enthalten zusammen mit der aktiven Verbindung Benetzungsmittel, Süßungsmittel, Färbemittel und Geschmacksstoffe. Verschiedene flüssige und pulverförmige Zusammensetzungen können durch herkömmliche Verfahren auch für die Inhalation vorbereitet werden. Tablettenzusammensetzungen können auch unter Verwendung von Verfahren nach dem Stand der Technik hergestellt werden.

Injizierbare Zusammensetzungen können verschiedene Träger wie pflanzliche Öle, Dimethylacetamid, Dimethylformamid, Ethyllactat, Ethylcarbonat, Isopropylmyristat, Ethanol, Polyole (Glycerin, Propylenglykol, flüssiges Polyethylenglykol und dergleichen) enthalten. Zur intravenösen Injektion können die Verbindungen (I und II) durch ein Tropfverfahren, bei denen die Verbindungen (I und II) mit einem physiologisch verträglichen Hilfsstoff infundiert wird, verabreicht werden. Physiologisch verträgliche Hilfsstoffe enthalten, beispielsweise 5% Dextrose, 0,9% Salzlösung, Ringer-Lösung oder einen anderen geeigneten Trägerstoff. Für die intramuskuläre Präparationen kann eine sterile Zusammensetzung einer geeigneten löslichen Salzform der Verbindung (I) und (II) gelöst und in einem pharmazeutischen Trägerstoff verabreicht werden, wie Wasser zur Injektion, 0,9% Kochsalzlösung oder 5% Glukoselösung oder Depotformen der Verbindungen, die beispielsweise Decanoat, Palmitat, Undecylensäure, Enanthat enthalten können.

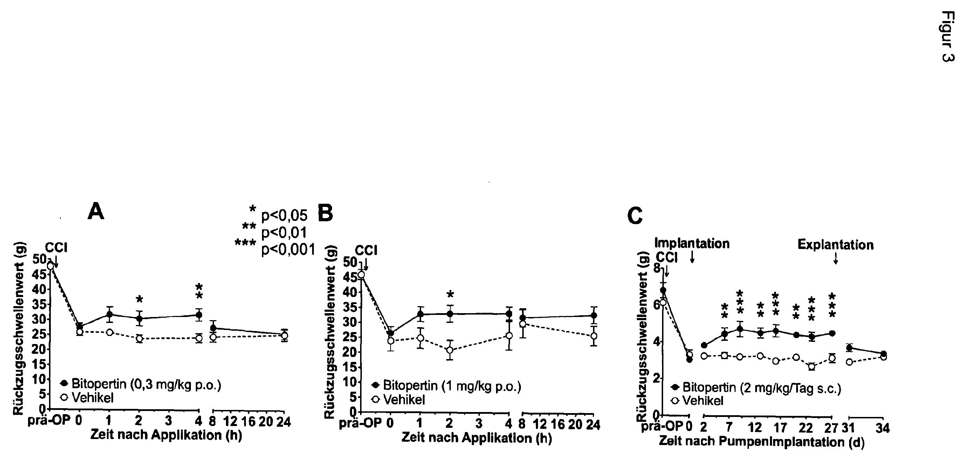

Zum Beweis der Effektivität der Bitopertin-Applikation zur Behandlung neuropathischer Schmerzen wurde eine operative Ligatur des linken *N. ischiadicus* (*Chronic Constriction Injury*; CCI) der Wistar Ratte sowie C57BL/6-Maus induziert. Am Tag 10-12 nach CCI erhielten 2 Gruppen der Ratten randomisiert und verblindet eine einmalige orale Dosierungen von Bitopertin (0,3; 1 mg/kg), während die Kontrollgruppen Vehikel erhielten. Mechanische Allodynie wurde mittels eines halbautomatischen Frey Filaments (Ugo Basile) vor und 10-12 Tage nach CCI, sowie 1, 2, 4, 8 und 24 h nach Applikation von Bitopertin bzw. Vehikel überprüft. Zusätzlich wurde die Wirkung von Bitopertin 10 Tage nach CCI in Mäusen bei kontinuierlicher Langzeitapplikation mittels subkutan implantierter, osmotischer Mini-Infusionspumpen (2 mg/kg/Tag) über 27 Tage untersucht. Neuromotorische Wirkungen von Bitopertin in Mäusen wurden im Open-Field-Test 1 h nach Injektion bzw. bei kontinuierlicher Applikation nach 12 Tagen analysiert. Statistik: Alle Daten sind dargestellt als Mittelwerte ± Standardfehler; ANOVA und Bonferroni post hoc Test; Signifikanzniveau p<0,05. Als Ergebnis führte Bitopertin (0,3 mg/kg) bei Ratten zwei und vier Stunden nach oraler Applikation zu einer Reduktion der durch CCI induzierten mechanischen Allodynie, wie auch aus Figur 3 in Abbildung A zu erkennen ist. Eine höhere Dosis von 1 mg/kg Bitopertin führte zu vergleichbar ausprägten Effekten (vergleiche Figur 3 mit Abbildung B). Die kontinuierliche subkutane Applikation von Bitopertin in Mäusen (2 mg/kg/Tag) nach CCI führte 7 Tagen nach Beginn bis zur Beendigung der Therapie zu einer verminderten Schmerzempfindlichkeit der neuropathischen Extremität der Mäuse wie aus Figur 3 Abbildung C zu erkennen ist. Es wurden keine Unterschiede in Wegstrecken und Aufenthaltsdauern nach einmaliger Gabe von Bitopertin (2 mg/kg s.c.) und kontinuierlicher Applikation (2 mg/kg/Tag s.c.) in naiven Mäusen und nach CCI im Vergleich zu Kontrolltieren nach Erhalt von NaCl 0,9% beobachtet (je n=4).

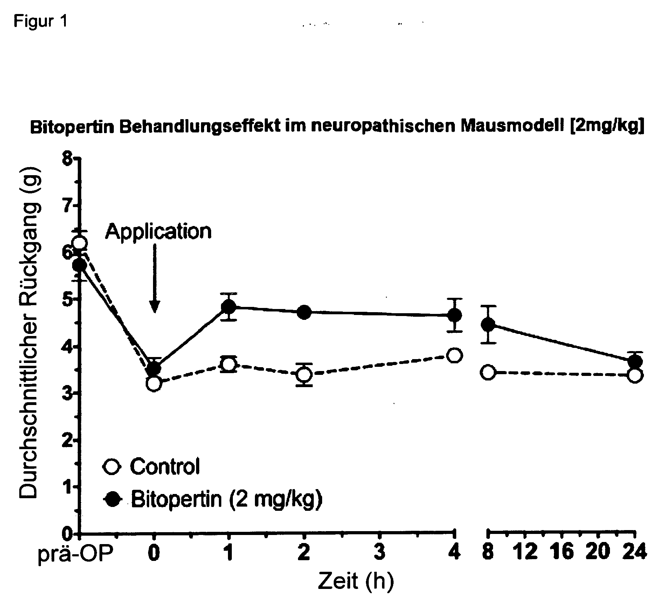

Die Erfindung wird anhand der Figuren 1 bis 3 nochmals eingehend in ihrer Anwendung beschrieben.

Figur 1 zeigt ein Modell zur Applikation des GlyT1-Inhibitors Bitopertin als Medikament zur Behandlung peripherer neuropathischer Schmerzen. Dabei wurde an Mäusen eine Applikation vorgenommen, wobei eine intraperitoneale Applikation mit einer Dosis von 2 mg/kg gegenüber der Verabreichung eines Kontroll-Standards (Kochsalzlösung 0,9%) dargestellt ist. Es zeigte sich eine deutliche Anhebung der gemessenen Rückzugsschwellenwerte in Antwort auf eine mechanische Stimulation der neuropathischen Extremität. Dies ist Ausdruck einer Minderung der bei diesen Tieren bestehenden neuropathischen Symptomatik einer mechanischen Allodynie.

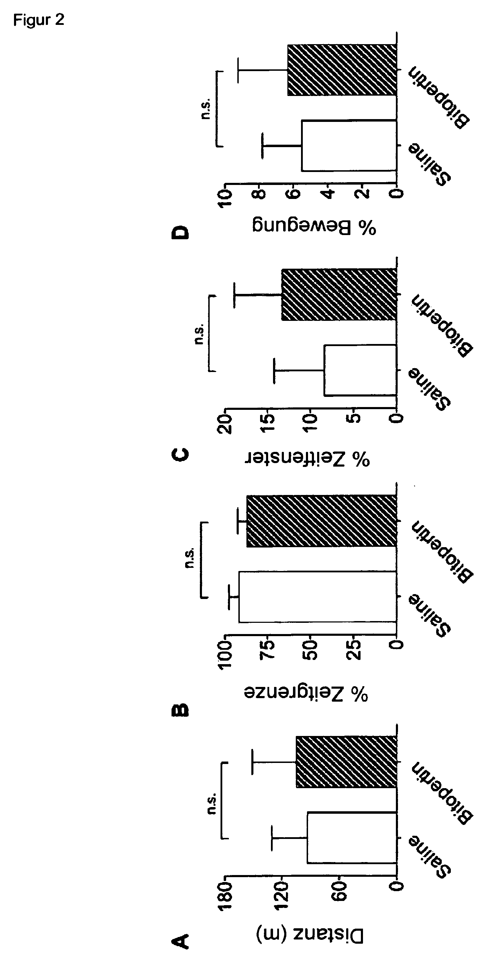

Figur 2 zeigt zusätzliche Verhaltensexperimente (Open-field Test) mit Bitopertinbehandelten Mäusen nach Gabe von Bitopertin (2 mg/kg), wobei im Vergleich zu Placebo-Behandlung keine Veränderungen der Aktivität festzustellen ist.

In Figur 3 ist der Effekt des GlyT1-Inhibitors Bitopertin auf die mechanische Allodynie bei Nagern mit Neuropathie nach CCI dargestellt. Abbildung (A) zeigt den Effekt des GlyT1-Inhibitors Bitopertin auf die einmalige orale Applikation bei Ratten 0,3 mg/kg; n=9-11. Abbildung (B) zeigt den Effekt des GlyT1-Inhibitors Bitopertin auf die einmalige orale Applikation bei Ratten 1 mg/kg; n=6-13; Vehikel: 0,3% Polysorbat 80 und 0,02 bzw. 0,06 % DMSO. Abbildung (C) zeigt schließlich den Effekt des GlyT1-Inhibitors Bitopertin bei kontinuierlicher subkutaner Applikation bei Mäusen; n=4; Vehikel: NaCl 0,9%; *p<0,05; **p<0,01; ***p<0,001. Es aus Figur 3 zu erkennen, dass die Applikation des GlyT1-Inhibitors Bitopertin nach einmaliger sowie kontinuierlicher Gabe im Tiermodell für neuropathischen Schmerz zu einer signifikanten Verminderung von mechanischer Allodynie führt. Es kommt dabei nicht zu unerwünschten neuromotorischen Wirkungen.

## Patentansprüche

1. Die Verwendung einer Verbindung nach der Formel (I) 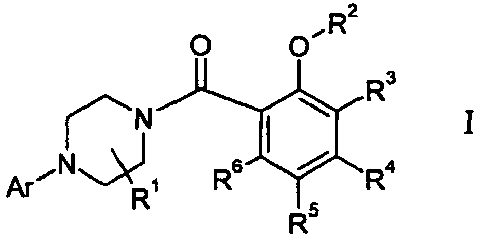 worin,
Ar unsubstituiertes oder substituiertes Aryl, ausgewählt aus der Gruppe, bestehend aus Phenyl, Benzyl, Naphthyl, Biphenyl und Indanyl, oder 6-gliedriges Heteroaryl ist, enthaltend ein, zwei oder drei Stickstoffatome, und wobei die Aryl- und die Heteroarylgruppen durch einen oder mehrere Substituenten, ausgewählt aus der Gruppe, bestehend aus Hydroxy, Halogen, NO₂, CN, (C₁-C₆)-Alkyl, (C₁-C₆)-Alkyl, substituiert durch Halogen, (C₁-C₆)-Alkyl, substituiert durch Hydroxy, (CH₂)ₙ-(C₁-C₆)-Alkoxy, (C₁-C₆)-Alkoxy, substituiert durch Halogen, NR₇R₈, C(O)R₉, SO₂R₁₀, -C(CH₃)=NOR₇, oder durch einen 5-gliedrigen aromatischen Heterozyklus, enthaltend 1 bis 4 Heteroatome, ausgewählt aus N und O, der gegebenenfalls durch (C₁-C₆)-Alkyl substituiert ist, substituiert sein können;
R₁ = Wasserstoff oder (C₁-C₆)-Alkyl ist;
R₂ = Wasserstoff, (C₁-C₆)-Alkyl, (C₂-C₆)-Alkenyl, (C₁-C₆)-Alkyl, substituiert durch Halogen, (C₁-C₆)-Alkyl, substituiert durch Hydroxy, (CH₂)ₙ-(C₃-C₇)-Cycloalkyl, gegebenenfalls substituiert durch (C₁-C₆)-Alkoxy oder durch Halogen, ist oder CH(CH₃)-(C₃-C₇)-Cycloalkyl, (CH₂)ₙ₊₁-C(O)-R₉, (CH₂)ₙ₊₁-CN, Bicyclo [2.2.1]heptyl, (CH₂)ₙ₊₁-O-(C₁-C₆)-Alkyl, (CH₂)ₙ-Heterocycloalkyl, (CH₂)ₙ-Aryl oder (CH₂)ₙ-Heteroaryl mit 5 oder 6 Ringgliedern, enthaltend ein, zwei oder drei Heteroatome, ausgewählt aus der Gruppe, bestehend aus Sauerstoff, Schwefel oder Stickstoff, ist, worin Aryl, Heterocycloalkyl und Heteroaryl unsubstituiert oder durch einen oder mehrere Substituenten substituiert sind, ausgewählt aus der Gruppe, bestehend aus Hydroxy, Halogen, (C₁-C₆)-Alkyl oder (C₁-C₆)-Alkoxy;
R₃, R₄ und R₆ unabhängig voneinander = Wasserstoff, Hydroxy, Halogen, (C₁-C₆)-Alkyl, (C₁-C₆)-Alkoxy oder O-(C₃-C₆)-Cycloalkyl sind;
R₅ = NO₂, CN, C(O)R₉ oder SO₂ R₁₀ ist;
R₇ und R₈ unabhängig voneinander = Wasserstoff oder (C₁-C₆)-Alkyl sind;
R₉ = Wasserstoff, (C₁-C₆)-Alkyl, (C₁-C₆)-Alkoxy oder NR₇R₈ ist;
R₁₀ = (C₁-C₆)-Alkyl, gegebenenfalls substituiert durch Halogen, (CH₂)ₙ-(C₃-C₆) Cycloalkyl, (CH₂)ₙ-(C₃-C₆)-Alkoxy, (CH₂)ₙ-Heterocycloalkyl oder NR₇R₈ ist;
n =0, 1 oder 2 ist;
und pharmazeutisch akzeptable Säureadditionssalze davon, als Glycin-Transporter-1-Antagonist in der Herstellung eines Medikaments zur Behandlung chronischer und/oder neuropathischer Schmerzen.

2. Die Verwendung einer Verbindung der Formel (I) in der Herstellung eines Medikaments zur Behandlung chronischer und/oder neuropathischer Schmerzen nach Anspruch 1, worin
Ar substituiertes Phenyl, eine substituierte 6-gliedrige Heteroarylgruppe ist, enthaltend ein, zwei oder drei Stickstoffatome,
R₂ = (C₁-C₆)-Alkyl, durch Halogen substituiertes (C₁-C₆)-Alkyl, (CH₂)ₙ-(C₃-C₇)-Cycloalkyl oder Bicyclo [2.2.1]-heptyl, (CH₂)ₙ-O-(C₁-C₆)-Alkyl oder (CH-Heterocycloalky) ist und
R₅ = S(O)₂CH₃,S(O)₂CH₂CH₃, S(O)₂NHCH₃ oder NO₂ ist.

3. Die Verwendung einer Verbindung nach Anspruch 1 oder 2 zur Herstellung eines Medikaments zur Behandlung chronischer und/oder neuropathischer Schmerzen, insbesondere der Formel (II)

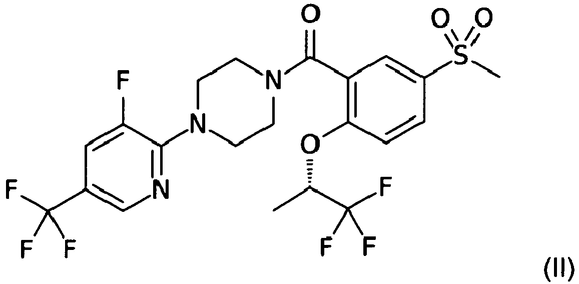

4. Die Verwendung einer Verbindung der Formel (I) in der Herstellung eines Medikaments zur Behandlung chronischer und/oder neuropathischer Schmerzen nach Anspruch 1 bis 3, wobei mindestens ein Bestandteil des Medikaments ein Analgetikum, insbesondere ein Opiat wie Oxycodon, umfasst.

5. Die Verwendung einer Verbindung der Formel (I) in der Herstellung eines Medikaments zur Behandlung chronischer und/oder neuropathischer Schmerzen nach Anspruch 1 bis 4, wobei mindestens ein Bestandteil des Medikaments ein Antidepressivum, insbesondere Amitriptylin umfasst.

6. Die Verwendung einer Verbindung der Formel (I) in der Herstellung eines Medikaments zur Behandlung chronischer und/oder neuropathischer Schmerzen nach Anspruch 1 bis 5, wobei mindestens ein Bestandteil des Medikaments ein Antikonvulsivum, insbesondere Gabapentin umfasst.

7. Die Verwendung einer Verbindung nach der Formel (I) in der Herstellung eines Medikaments zur Behandlung chronischer und/oder neuropathischer Schmerzen nach Anspruch 1 bis 6, wobei das Medikament oral (enteral) verabreichbar ist.

8. Die Verwendung einer Verbindung nach der Formel (I) in der Herstellung eines Medikaments zur Behandlung chronischer und/oder neuropathischer Schmerzen nach Anspruch 1 bis 7, wobei das Medikament (parenteral) injizierbar ist.

9. Die Verwendung einer Verbindung nach der Formel (I) in der Herstellung eines Medikaments zur Behandlung chronischer und/oder neuropathischer Schmerzen nach Anspruch 1 bis 8, wobei das Medikament eine Verbindung der Formel I in einer Menge von 0,1 bis 10000mg, insbesondere 1 bis 1000mg enthält.

10. Verfahren zur Behandlung chronischer und/oder neuropathischer Schmerzen umfassend das Verabreichen einer therapeutisch wirksamen Menge einer Verbindung der Formel (I), 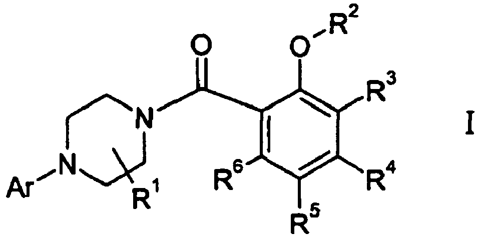 worin,
Ar unsubstituiertes oder substituiertes Aryl, ausgewählt aus der Gruppe, bestehend aus Phenyl, Benzyl, Naphthyl, Biphenyl und Indanyl, oder 6-gliedriges Heteroaryl ist, enthaltend ein, zwei oder drei Stickstoffatome, und wobei die Aryl- und die Heteroarylgruppen durch einen oder mehrere Substituenten, ausgewählt aus der Gruppe, bestehend aus Hydroxy, Halogen, NO₂, CN, (C₁-C₆)-Alkyl, (C₁-C₆)-Alkyl, substituiert durch Halogen, (C₁-C₆)-Alkyl, substituiert durch Hydroxy, (CH₂)ₙ-(C₁-C₆)-Alkoxy, (C₁-C₆)-Alkoxy, substituiert durch Halogen, NR₇R₈, C(O)R₉, SO₂R₁₀,-C(CH₃)=NOR₇, oder durch einen 5-gliedrigen aromatischen Heterozyklus, enthaltend 1 bis 4 Heteroatome, ausgewählt aus N und O, der gegebenenfalls durch (C₁-C₆)-Alkyl substituiert ist, substituiert sein können;
R₁ = Wasserstoff oder (C₁-C₆)-Alkyl ist;
R₂ = Wasserstoff, (C₁-C₆)-Alkyl, (C₂-C₆)-Alkenyl, (C₁-C₆)-Alkyl, substituiert durch Halogen, (C₁-C₆)-Alkyl, substituiert durch Hydroxy, (CH₂)ₙ-(C₃-C₇)-Cycloalkyl, gegebenenfalls substituiert durch (C₁-C₆)-Alkoxy oder durch Halogen, ist oder CH(CH₃)-(C₃-C₇)-Cycloalkyl, (CH₂)ₙ₊₁-C(O)-R₉, (CH₂)ₙ₊₁-CN, Bicyclo [2.2.1]heptyl, (CH₂)ₙ₊₁-O-(C₁-C₆)-Alkyl, (CH₂)ₙ-Heterocycloalkyl, (CH₂)ₙ-Aryl oder (CH₂)ₙ-Heteroaryl mit 5 oder 6 Ringgliedern, enthaltend ein, zwei oder drei Heteroatome, ausgewählt aus der Gruppe, bestehend aus Sauerstoff, Schwefel oder Stickstoff, ist, worin Aryl, Heterocycloalkyl und Heteroaryl unsubstituiert oder durch einen oder mehrere Substituenten substituiert sind, ausgewählt aus der Gruppe, bestehend aus Hydroxy, Halogen, (C₁-C₆)-Alkyl oder (C₁-C₆)-Alkoxy;
R₃, R₄ und R₆ unabhängig voneinander = Wasserstoff, Hydroxy, Halogen, (C₁-C₆)-Alkyl, (C₁-C₆)-Alkoxy oder O-(C₃-C₆)-Cycloalkyl sind;
R₅ = NO₂, CN, C(O)R₉ oder SO₂ R₁₀ ist;
R₇ und R₈ unabhängig voneinander = Wasserstoff oder (C₁-C₆)-Alkyl sind;
R₉ = Wasserstoff, (C₁-C₆)-Alkyl, (C₁-C₆)-Alkoxy oder NR₇R₈ ist;
R₁₀ = (C₁-C₆)-Alkyl, gegebenenfalls substituiert durch Halogen, (CH₂)ₙ-(C₃-C₆) Cycloalkyl, (CH₂)ₙ-(C₃-C₆)-Alkoxy, (CH₂)ₙ-Heterocycloalkyl oder NR₇R₈ ist;
n =0, 1 oder 2 ist.

11. Verfahren zur Behandlung chronischer und/oder neuropathischer Schmerzen nach Anspruch 10, umfassend einen Schritt des Co-Verabreichens mindestens eines Analgetikums.

12. Verfahren zur Behandlung chronischer und/oder neuropathischer Schmerzen nach Anspruch 10 oder 11, wobei die co-verabreichten Analgetika, insbesondere ein Opiat, insbesondere Oxycodon umfassen.

13. Verfahren zur Behandlung chronischer und/oder neuropathischer Schmerzen nach einem der Ansprüche 10 bis 12, wobei das Medikament oral (enteral) applizierbar ist.

14. Verfahren zur Behandlung chronischer und/oder neuropathischer Schmerzen nach einem der Ansprüche 10 bis 13, wobei das Medikament (parenteral) injizierbar ist.

15. Verfahren zur Behandlung chronischer und/oder neuropathischer Schmerzen nach einem der Ansprüche 10 bis 14, wobei das Medikament einem menschlichen Individuum in einer Menge von 0,1 bis 10000 mg, insbesondere 1 bis 1000 mg pro Tag verabreicht wird.
